# FASCICULE DE BREVET EUROPEEN

(11) **EP 4 456 981 B1**
(45) Date de publication et mention de la délivrance du brevet: **10.09.2025**
(21) Numéro de dépôt: 22844506.0
(22) Date de dépôt: 28.12.2022
(51) Int. Cl.: A61N 7/00, A61B 8/00, A61B 18/00, A61B 90/00, A61B 8/08

(54) **SYSTEME POUR LA DETECTION D'UNE BORNE DE CONNEXION**
SYSTEM ZUR ERKENNUNG EINER ANSCHLUSSKLEMME
SYSTEM FOR DETECTING A CONNECTION TERMINAL

(30) Priorité: 28.12.2021 FR 2114574
(43) Date de publication de la demande: 06.11.2024
(73) Titulaire: Carthera, 69008 Lyon (FR)
(72) Inventeur: BOUCHOUX, Guillaume, 69100 VILLEURBANNE (FR); CHOLVY, Matthieu, 38550 PEAGE DE ROUSSILLON (FR)
(74) Mandataire: Be IP
(86) Numéro de dépôt international: PCT/EP2022/087934
(87) Numéro de publication internationale: WO 2023/126430

(56) Documents cités:
- WO-A1-2018/007500
- US-A- 5 904 691
- US-A1- 2003 187 351
- US-A1- 2020 138 580
- US-A1- 2020 139 159

## Description

Le projet menant au dépôt de cette demande a reçu un financement du programme de recherche et d'innovation Horizon 2020 de l'Union européenne dans le cadre de la convention de subvention n° 960141.

### DOMAINE DE L'INVENTION

La présente invention concerne le domaine technique général des dispositifs de traitement (notamment ultrasonores) - par exemple des dispositifs intracorporels ou implantables - destinés à être raccordés électriquement à une unité de commande distante.

De tels dispositifs peuvent notamment être implantés chez l'homme et les mammifères pour traiter une pathologie, tel qu'un glioblastome en utilisant des ultrasons.

### ARRIERE PLAN DE L'INVENTION

On connaît du document WO 2018/007500 un appareil de traitement d'affections du cerveau.

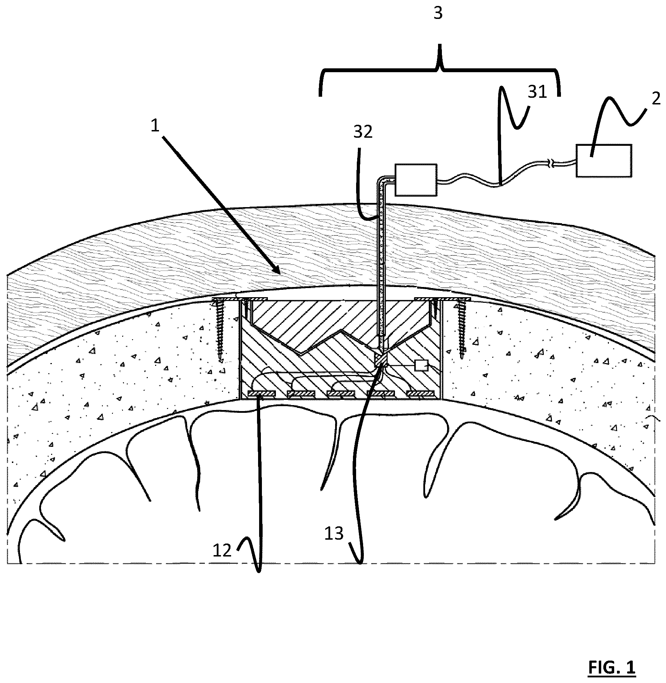

En référence à la figure 1, un tel appareil se compose :
- d'un dispositif ultrasonore 1 réalisé en matériau non ferromagnétique,
- d'une unité de commande 2 distante du dispositif ultrasonore 1, et
- de moyens de connexion 3 entre le dispositif ultrasonore 1 et l'unité de commande 2.

Le dispositif ultrasonore 1 est destiné à être positionné dans un trou de trépan effectué dans un crâne d'un patient. Il comprend :
- un support,
- un (ou plusieurs) transducteur(s) 12 pour la génération d'ondes ultrasonores de traitement d'une affection cérébrale,
- une (ou plusieurs) borne(s) 13 de connexion électrique destinée(s) à coopérer avec les moyens de connexion 3.

L'unité de commande 2 est destinée à alimenter en énergie électrique le dispositif ultrasonore 1, et à régler ses paramètres de fonctionnement.

Les moyens de connexion 3 sont destinés à relier électriquement le dispositif ultrasonore 1 à l'unité de commande 2. Ils comprennent généralement :
- un (ou plusieurs) câble(s) 31 de connexion électrique dont l'une des extrémités est reliée à l'unité de commande, et
- une (ou plusieurs) aiguille(s) transdermique(s) 32 raccordée(s) à l'autre extrémité du câble 31.

Le principe de fonctionnement de cet appareil est le suivant. Une fois le dispositif ultrasonore 1 implanté dans le crâne du patient, une succession de séances de traitement lui sont prodiguées pour traiter la pathologie qui l'affecte. A chaque nouvelle séance de traitement, le dispositif ultrasonore 1 est relié à l'unité de commande 2 par l'intermédiaire des moyens de connexion 3.

Un personnel soignant relie le câble 31 à l'unité de commande 2 puis il insère l'aiguille 32 à travers la peau du patient jusqu'à la borne 13 du dispositif ultrasonore.

Une fois l'extrémité de l'aiguille 32 connectée à la borne 13, l'unité de commande 2 peut être activée pour alimenter le dispositif ultrasonore 1 en énergie électrique.

Actuellement, la borne 13 du dispositif ultrasonore 1 est détectée par palpation à travers le cuir chevelu du patient, car ce dernier a été suturé en place par le chirurgien après l'implantation.

Toutefois, chez certains patients, la détection de la borne 13 peut s'avérer difficile du fait de différents facteurs (œdème, épaisseur importante du cuir chevelu, etc.). Ceci augmente la durée nécessaire pour raccorder électriquement l'aiguille 32 à la borne 13c (durée supérieure à deux minutes). En outre, plusieurs tentatives d'insertion peuvent être nécessaire, ce qui augmente l'inconfort pour le patient.

Un but de la présente invention est de proposer un procédé et un système permettant au personnel soignant de faciliter la détection de la position d'une borne de connexion d'un dispositif ultrasonore implanté.

### BREVE DESCRIPTION DE L'INVENTION

L'invention est définie par la revendication 1. D'autres modes de mise en œuvre de l'invention sont définis dans les revendications dépendantes.

A cet effet, l'invention propose un appareil de traitement d'une pathologie comprenant :
- un dispositif implantable au niveau d'une ouverture ménagée dans la boîte crânienne d'un patient, le dispositif implantable comportant une unité de traitement ayant des faces supérieure et inférieure et incluant une borne de connexion électrique s'étendant sur la face supérieure,
- une unité de commande distante pour déterminer et contrôler des paramètres de fonctionnement du dispositif implantable, et lui délivrer de l'électricité,
- des moyens de connexion électrique pour le raccordement électrique de l'unité de traitement à l'unité de commande distante par l'intermédiaire de la borne de connexion électrique,
***remarquable en ce que*** le dispositif implantable comprend un repère de positionnement pour faciliter la détection de la position de la borne de connexion électrique, ledit repère de positionnement comprenant au moins deux sources lumineuses, telles que des diodes électroluminescentes, aptes à émettre chacune un rayonnement lumineux vers l'extérieur de la face supérieure, la borne de connexion électrique s'étendant entre lesdites diodes électroluminescentes.

Des aspects préférés mais non limitatifs de la présente invention sont les suivants :
- la longueur d'onde du rayonnement lumineux émis par chaque diode électroluminescente peut être comprise entre 600 et 1600 nanomètres, préférentiellement comprise entre 850 et 1250 nanomètres, et encore plus préférentiellement compris entre 950 et 1100 nanomètres, notamment de l'ordre de 1050 nanomètres ;
- la distance entre les diodes électroluminescentes peut être comprise entre 15 et 60 millimètres, de préférence comprise entre 20 et 40 millimètres, notamment de l'ordre de 25 millimètres ;
- la distance entre chaque diode électroluminescente et la borne de connexion peut être comprise entre 9 et 35 millimètres, de préférence comprise entre 12 et 25 millimètres, notamment de l'ordre de 15 millimètres;
- chaque diode électroluminescente peut être configurée pour émettre un rayonnement lumineux ayant une longueur d'onde respective différente des longueurs d'onde des autres diodes électroluminescentes ;
- chaque diode électroluminescente peut être configurée pour émettre un rayonnement lumineux ayant une intensité respective différente des intensités des autres diodes électroluminescentes ;
- le repère de positionnement peut comprendre en outre au moins un circuit électrique passif résonnant ;
- ledit et au moins un circuit électrique passif résonnant peut être configuré pour interagir avec un circuit électrique résonant actif intégré à une unité de localisation, ladite interaction permettant d'alimenter le dispositif médical implantable en énergie électrique par induction ;
- ledit et au moins un circuit électrique passif résonnant peut comprendre une bobine fonctionnellement couplée à un condensateur, ladite bobine s'étendant autour de la borne de connexion électrique ;
- ledit et au moins un circuit électrique passif résonnant peut être configuré de sorte que la fréquence de résonnance dudit et au moins un circuit électrique passif résonnant est comprise entre 10MHz et 50 MHz ;
- le dispositif implantable peut comprendre :
   ∘ une plaque support incluant des première et deuxième faces opposées, l'unité de traitement étant destinée à être montée sur la première face de la plaque support,
   ∘ une pièce de fixation destinée à être positionnée sur la deuxième face de la plaque support et étant configurée pour plaquer l'unité de traitement contre la première face de la plaque support lorsque l'unité de traitement, la plaque support et la pièce de fixation sont assemblées,
   la pièce de fixation incluant le repère de positionnement ;
- avantageusement :
   ∘ la plaque support peut comprendre un orifice traversant,
   ∘ la borne de connexion électrique peut comprendre un pion s'étendant en saillie vers l'extérieur de l'unité de traitement, le pion étant destiné à être positionné dans l'orifice traversant de la plaque support,
   ∘ la pièce de fixation peut comprendre :
      ▪ un conduit adapté pour recevoir au moins une portion du pion, et
      ▪ une collerette périphérique s'étendant perpendiculairement à un axe longitudinal du conduit, la collerette étant destinée à être positionnée sur la deuxième face de la plaque support,
   la collerette périphérique incluant le repère de positionnement.

### BREVE DESCRIPTION DES DESSINS

D'autres avantages et caractéristiques du procédé et du système selon l'invention ressortiront mieux de la description qui va suivre de plusieurs variantes d'exécution, données à titre d'exemples non limitatifs, à partir des dessins annexés sur lesquels :
- la figure 1 illustre schématiquement un exemple d'appareil de traitement d'une affection cérébrale incluant un dispositif ultrasonore raccordé électriquement à une unité de commande distante grâce à des moyens de connexion (aiguille transdermique + câble),
- la figure 2 est une représentation schématique en coupe d'un d'appareil de traitement selon l'invention,
- la figure 3 est une représentation schématique en perspective d'un dispositif médical implantable,
- la figure 4 est une représentation schématique d'une unité ultrasonore du dispositif médical implantable,
- la figure 5 est une représentation schématique d'une plaque support du dispositif médical implantable,
- la figure 6 est une représentation schématique en perspective d'une pièce de fixation du dispositif médical implantable,
- la figure 7 est une image représentant un rayonnement lumineux émis à travers un fantôme simulant le comportement d'un cuir chevelu,
- la figure 8 est une image de trois rayonnement lumineux émis à travers le fantôme simulant le comportement du cuir chevelu,
- la figure 9 est une courbe illustrant l'intensité lumineuse d'un rayonnement émis à travers le fantôme simulant le comportement du cuir chevelu en fonction de l'épaisseur dudit fantôme,
- la figure 10 est une représentation schématique de circuits électroniques intégrés dans une unité de localisation d'une part et dans le dispositif médical implantable (et plus précisément dans une collerette du dispositif médical implantable).

### DESCRIPTION DETAILLEE DE L'INVENTION

On va maintenant décrire différents exemples du système et du procédé selon l'invention en référence aux figures. Dans ces différentes figures, les éléments équivalents sont désignés par la même référence numérique.

### 1. Généralités

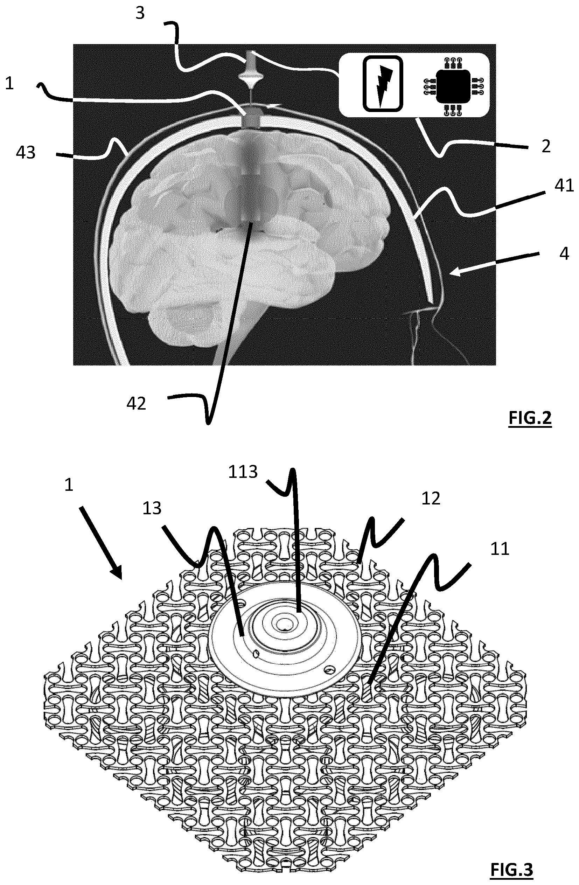

En référence à la figure 2, l'appareil de traitement comprend :
- un dispositif médical implantable 1 dans un patient 4,
- une unité de commande 2 distante pour déterminer et contrôler des paramètres de fonctionnement du dispositif implantable 1, et lui délivrer de l'électricité,
- des moyens de connexion électrique 3 pour le raccordement électrique du dispositif implantable 1 à l'unité de commande 2 distante,
- une unité de localisation du dispositif implantable 1.

Le dispositif médical 1 est apte à être implanté dans un os crânien 41 du patient 4 pour permettre le traitement et/ou l'imagerie d'une zone cérébrale d'intérêt 42. Pour ce faire, le praticien réalise une craniectomie. Une incision est faite dans le cuir chevelu 43, puis la peau (et les muscles le cas échéant) est soulevée (sont soulevés) afin d'exposer le crâne 41. Le crâne 41 est ensuite découpé pour former un volet osseux. Le volet osseux crânien est retiré pour laisser place à une ouverture crânienne dans laquelle le dispositif médical implantable 1 peut être positionné. Une fois le dispositif médical implantable 1 correctement positionné, celui-ci est fixé sur la périphérie de l'ouverture crânienne par tout moyen connu de l'homme du métier (vis d'ancrage, collage, etc.), puis le cuir chevelu 43 (peau et muscles) est remis en place pour recouvrir le dispositif médical implantable 1.

A chaque nouvelle séance de traitement/d'imagerie, un personnel soignant raccorde électriquement le dispositif médical implantable 1 à l'unité de commande 2 distante en utilisant les moyens de connexion 3.

Comme illustré à la figure 1, ces moyens de connexion 3 comprennent notamment :
- un câble 31 électriquement conducteur,
- une aiguille 32 montée à l'une des extrémités du câble 31, l'aiguille 32 étant apte à être introduite dans une borne de connexion 113 de l'unité ultrasonore 11, et
- une prise de liaison (non représentée) à l'autre des extrémités du câble 31, la prise de liaison étant apte à être connectée à une prise complémentaire de l'unité de commande 2.

Plus précisément, le personnel soignant connecte la prise de liaison à l'unité de commande 2 distante. Le personnel soignant insère ensuite l'aiguille 32 dans le cuir chevelu 43 du patient 4, et introduit l'extrémité de l'aiguille dans une borne de connexion 113 (illustré à la figure 3) de sorte à finaliser le raccordement électrique du dispositif médical implantable 1 à l'unité de commande 2 distante.

Il peut être difficile pour le personnel soignant d'identifier par palpation la position de la borne de connexion 113 une fois le dispositif médical 1 implanté, celui-ci étant recouvert par le cuir chevelu 43 (peu et muscles) du crâne du patient 4. Or, la connaissance de la position précise de la borne de connexion 113 est nécessaire pour assurer un positionnement adéquat de l'aiguille 32 transdermique.

Pour faciliter la détection de la borne de connexion 113 à travers le cuir chevelu 43 du patient 4, le dispositif médical implantable 1 comprend un repère de positionnement. Ce repère de positionnement est configuré pour interagir avec l'unité de localisation.

Cette unité de localisation permet de détecter la position précise de la borne de connexion 113.

Avantageusement, l'unité de localisation peut être contenue dans une pièce à main, ou être intégrée aux moyens de connexion 3.

### 2. Dispositif médical implantable

### 2.1. Présentation générale

En référence à la figure 3, le dispositif médical implantable 1 comprend :
- une unité ultrasonore 11 pour l'émission d'ondes ultrasonores d'imagerie ou de traitement,
- une plaque support 12 sur laquelle est montée l'unité ultrasonore 11, et
- une pièce de fixation 13 pour bloquer l'unité ultrasonore 11 contre la plaque support 12.

L'unité ultrasonore 11, la plaque support 12, et la pièce de fixation 13 sont des éléments distincts destinés à être assemblés pour former le dispositif médical implantable 1. Plus précisément pour former le dispositif médical implantable 1, ces différents éléments sont assemblés de sorte que la plaque support 12 s'étende entre l'unité ultrasonore 11 et la pièce de fixation 13.

Lorsque le dispositif médical implantable 1 est positionné dans l'ouverture crânienne, l'unité ultrasonore 11 s'étend en regard de la zone cérébrale d'intérêt 42. Ainsi, une fois implanté, l'unité ultrasonore 11 fait face à la zone cérébrale d'intérêt 42, tandis que la pièce de fixation 13 s'étend en regard du cuir chevelu 43 du patient 4.

Le repère de positionnement permet de faciliter la détection de la position de la borne de connexion 113 par le personnel soignant afin de faciliter l'insertion de l'aiguille 32 dans ladite borne de connexion 113.

Pour ce faire, le repère de positionnement comprend un (ou plusieurs) marqueur(s) entourant la borne de connexion 113. Ce (ou ces) marqueur(s) peuvent être du type marqueur optique et/ou marqueur à résonance électromagnétique, comme il sera décrit plus en détails dans la suite.

L'utilisation d'un repère de positionnement permet de réduire le temps nécessaire à la mise en œuvre d'une séance d'imagerie et/ou de traitement de la zone cérébrale 42, notamment par rapport à une solution basée sur l'utilisation d'un ensemble de neuro-navigation.

En effet, pour utiliser un ensemble de neuro-navigation, il peut être nécessaire de déplacer le patient 4, ce qui :
- est coûteux en temps,
- accroît les risques infectieux, et
- peut induire un stress supplémentaire chez le patient.

### 2.2. Unité ultrasonore

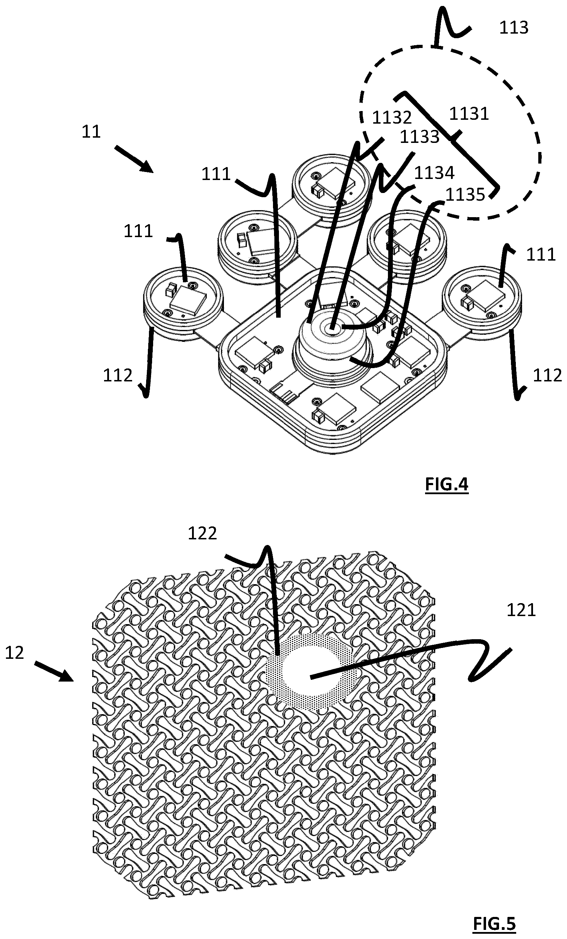

En référence à la figure 4, l'unité ultrasonore 11 comprend :
- une (ou plusieurs) carte(s) électronique(s) 111 adaptée(s) pour échanger des signaux électriques d'alimentation et de contrôle avec l'unité de commande 2 distante,
- un (ou plusieurs) transducteur(s) ultrasonore(s) 112 - par exemple circulaire(s) de 10 millimètres de diamètre (chacun) - adapté(s) pour générer des ondes ultrasonores de traitement (ou d'imagerie) de la zone cérébrale d'intérêt 42, et
- une borne de connexion électrique 113 pour le raccordement de l'unité ultrasonore 11 à l'unité de commande 2 distante.

La (ou les) carte(s) électronique(s) 111 et le (ou les) transducteur(s) 112 sont positionnés sur une première face de la plaque support 12 lorsque le dispositif médical implantable 1 est assemblé. La carte électronique 111 et les transducteurs 112 étant connus de l'homme du métier, ceux-ci ne seront pas décrits plus en détails dans la suite.

La borne de connexion électrique 113 permet de connecter le dispositif médical implantable 1 à l'unité de commande 2 externe qui alimente les transducteurs 112 en énergie électrique, et règle leurs paramètres de fonctionnement.

La borne de connexion 113 comprend un pion 1131 s'étendant en saillie vers l'extérieur d'une face supérieure de l'unité ultrasonore 11. La paroi supérieure 1132 du pion 1131 comporte un trou borgne 1133 dans lequel l'extrémité de l'aiguille 32 est destinée à être introduite pour raccorder électriquement l'unité ultrasonore 11 aux moyens de connexion électrique 3. Avantageusement, la borne de connexion 113 peut comprendre un évasement conique (ou fraisure) 1134 ménagé à l'entrée du trou borgne 1133. Ceci permet de guider l'aiguille 32 vers le trou borgne 1133 pour faciliter l'introduction de l'extrémité de l'aiguille dans le trou borgne 1133.

La paroi latérale du pion 1131 peut comprendre un filetage 1135. Ce filetage 1135 est destiné à coopérer par vissage avec un filetage correspondant ménagé sur la face interne d'un conduit de la pièce de fixation 13. Ceci permet d'assurer la solidarisation de l'unité ultrasonore 11, de la plaque support 12 et de la pièce de fixation 13 lors de l'assemblage du dispositif médical 1. Le fait que l'unité ultrasonore 11 coopère par vissage avec la pièce de fixation 13 permet, lors de la lors de la phase de raccordement du dispositif intracrânien à l'unité de commande, de répartir la force appliquée par l'aiguille sur la borne de connexion à toute une surface de la plaque support 12.

### 2.3. Plaque support

En référence à la figure 5, on a illustré un exemple de plaque support 12. La plaque support 12 est généralement rectangulaire, mais peut présenter une forme quelconque, telle qu'une forme circulaire, triangulaire ou carrée.

Le matériau constituant la plaque support 12 peut être un métal, tel que du titane ou tout autre métal connu de l'homme du métier (éventuellement recouvert de parylène ou équivalent si le métal utilisé n'est pas biocompatible en soi).

La plaque support 12 comprend un orifice traversant 121 pour le passage de la borne de connexion 113. Le bord 122 de l'orifice traversant 121 peut être recouvert d'une couche de matériau polymère, tel que de la silicone. Cette couche de matériau polymère permet de limiter les risques de desserrage entre la borne de connexion 113 et la pièce de fixation 13.

### 2.4. Pièce de fixation

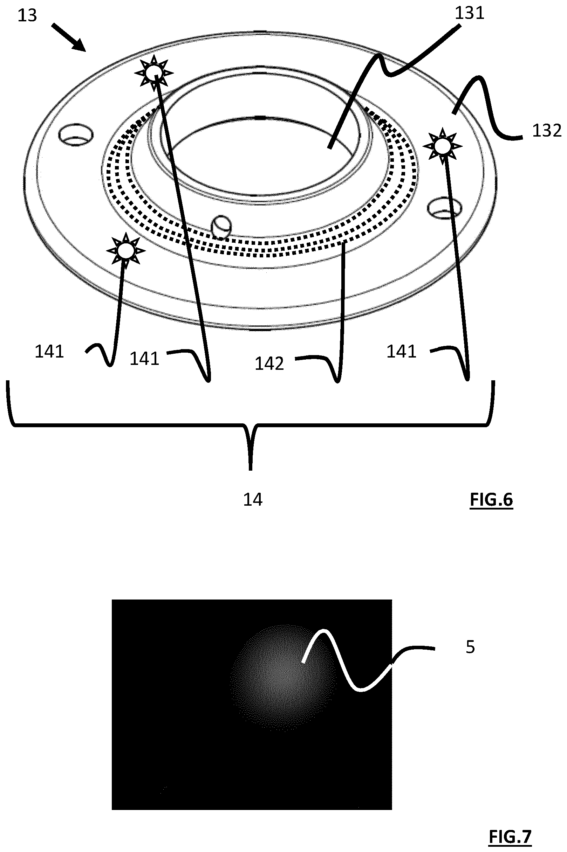

En référence à la figure 6, la pièce de fixation 13 comprend :
- un conduit 131 apte à recevoir le pion 1131, et
- une collerette 132 périphérique.

Le conduit 131 est destiné à coopérer avec la borne de connexion 113 de sorte à bloquer la plaque support 12 entre l'unité ultrasonore 11 et la pièce de fixation 13. Plus précisément, le conduit 131 consiste en un écrou dont le trou taraudé est destiné à coopérer par vissage avec le filetage 1135 de la paroi latérale du pion 1131. En d'autres termes, la face interne du conduit 131 comprend un filet complémentaire du filetage 1135 de la paroi latérale du pion 1131.

La collerette 132 s'étend à la base du conduit 131, perpendiculairement à l'axe de révolution du conduit 131. Elle est destinée à venir en contact avec une deuxième face de la plaque support 12 opposée à la première face en regard de la face supérieure de l'unité ultrasonore 11. La collerette 132 permet de plaquer la plaque support 12 contre l'unité ultrasonore 11 lorsque le dispositif médical implantable 1 est assemblé.

De préférence, la collerette 132 est de forme circulaire. Ceci permet une meilleure répartition de la force appliquée par l'aiguille lors de l'introduction de celle-ci dans le trou borgne 1143 de la borne de connexion114.

Le principe d'assemblage du dispositif implantable 1 est le suivant. Un opérateur insère le pion 1131 de la borne de connexion 113 à travers l'orifice traversant 122 de la plaque support 12. Une fois l'unité ultrasonore 11 en position sur la première face de la plaque support 12, l'opérateur dispose ensuite la pièce de fixation 13 sur la borne de connexion 113. La pièce de fixation 13 est installée sur le pion 1141 de sorte que la base du conduit 131 (au niveau de laquelle s'étend la collerette 132) soit en regard de la deuxième face de la plaque support 12 (opposée à la première face). L'opérateur visse ensuite la pièce de fixation 13 sur le pion 1131, ce qui induit le placage de la collerette 132 contre la plaque support 12 : l'unité ultrasonore 11, la plaque support 12 et la pièce de fixation sont alors solidaires. On obtient ainsi le dispositif médial implantable 1 illustré à la figure 2.

Avantageusement, et comme illustré à la figure 6, la collerette 132 peut comprendre le repère de positionnement permettant de faciliter la détection de la borne de connexion 113 par le personnel médical. Le fait que le repère de positionnement soit intégré à la pièce de fixation 13 permet de limiter les modifications à apporter au dispositif décrit dans WO 2018/007500 pour faciliter la détection de la borne de connexion 113.

On va maintenant décrire plus en détails différentes caractéristiques associées au repère de positionnement selon l'invention.

### 2.5. Repère de positionnement

Différentes solutions ont été proposées pour le repère de positionnement, parmi lesquelles :
- une solution optique dans laquelle le repère de positionnement comprend deux (ou plus de deux) sources lumineuses - telles que des diodes électroluminescentes (LED) - entourant la borne de connexion, et/ou
- une solution électromagnétique dans laquelle le repère de positionnement comprend un (ou plusieurs) circuit(s) électrique(s) passif(s) résonnant(s) entourant la borne de connexion.

Dans tous les cas, l'unité de localisation comprend un (ou plusieurs) capteur(s) adapté(s) pour interagir avec le repère de positionnement. Ce (ou ces) capteur(s) peut (peuvent) consister en :
- un détecteur de lumière - tel qu'une caméra - lorsque le repère de positionnement comprend des marqueurs optiques, et/ou
- un circuit résonnant lorsque le repère de positionnement comprend un (ou plusieurs) circuit(s) électrique(s) actif(s) résonnant(s).

Les avantages associés à chacune des solutions envisagées (optique/électromagnétique) pour le repère de positionnement vont maintenant être présentés en référence aux figures.

### 2.5.1. Solution optique

### 2.5.1.1. Principe

La solution optique utilise la détection de rayonnements lumineux émis par des sources lumineuses (telles que des LED) à travers le cuir chevelu du patient pour détecter la position de la borne de connexion.

Un (ou plusieurs) détecteur(s) de lumière - tel(s) qu'une (ou plusieurs) caméra(s) - est (sont) situé(s) dans l'unité de localisation (qui peut ou non être intégrée aux moyens de connexion) et reçoit (reçoivent) la lumière émise par les sources lumineuses.

En raison de la diffusion à travers les tissus du cuir chevelu, le rayonnement lumineux émis par chaque source lumineuse LED apparaît sous forme de « *tache* » 5 sur le (ou les) détecteur(s), comme illustré à la figure 7.

Le centre de chaque tache 5 correspond à la position de la source lumineuse ayant généré le rayonnement lumineux associé à ladite tache.

Pour déterminer la position de la borne de connexion 113 dans le cas d'un repère de positionnement incluant trois sources lumineuses situées chacune à égale distance de ladite borne de connexion 113, le principe est le suivant.

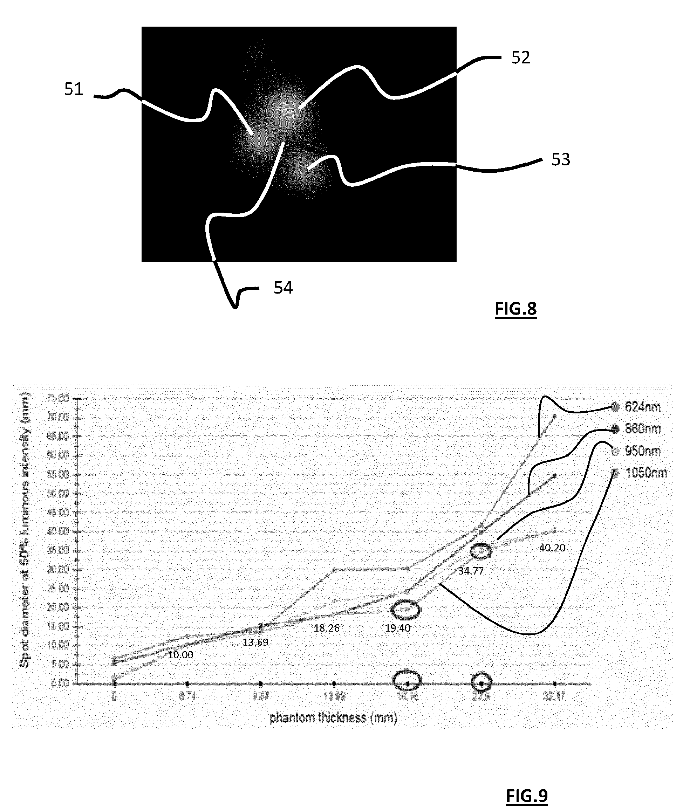

Un opérateur déplace l'unité de localisation sur le cuir chevelu du patient. Le(s) détecteur(s) acquiert (acquièrent) une (ou plusieurs) image(s) des rayonnements lumineux émis par les sources de lumière. Cette (ou ces images) peuvent être affichées sur des moyens d'affichage tels qu'un écran. Une telle image est représentée à la figure 8.

Le centre de chaque tache 51, 52, 53 est calculé - par un calculateur qui peut être intégré (ou non) à l'unité de localisation - pour estimer la position de chaque source lumineuse. En effet comme indiqué précédemment, le centre de chaque tache est représentatif de la position de la source lumineuse ayant produit la tache. Les centres ainsi calculés peuvent être affichés sur l'image (ou les images) affichée(s) sur les moyens d'affichage.

Le barycentre 54 des centres des taches 51-53 est ensuite calculé par le calculateur pour estimer la position de la borne de connexion. Plus précisément, le barycentre des centres des taches 51-53 est représentatif de la position de la borne de connexion 113.

Le fait d'utiliser 3 LED faisant chacune des spots larges et séparables, permet d'avoir une précision bien supérieure à la résolution de chaque spot.

Tout en surveillant la caméra, l'opérateur déplace l'aiguille des moyens de connexion au centre de la borne de connexion, et l'introduit dans le cuir chevelu du patient pour raccorder électriquement le dispositif médical 1 à l'unité de commande 2.

### 2.5.1.2. Expérimentation et résultats

Une expérience a été réalisée pour étudier :
- l'influence de l'épaisseur du cuir chevelu, et
- l'effet de la longueur d'onde du rayonnement lumineux émis par chaque source lumineuse.

Pour cette expérience, une diode électroluminescente a été insérée dans un fantôme d'agar imitant le comportement optique du cuir chevelu. Ce fantôme d'agar était composé :
- d'un litre d'eau,
- de 20g de gélose en poudre,
- de 1,67 g de dioxyde de titane (TiO₂),
- de 0,19 ml d'encre de Chine, et de
- de 1 g d'acide benzoïque.

Une caméra PiNoIR Raspberry Pi (émulant le comportement de l'unité de localisation) a été utilisée pour capturer la lumière ayant traversée le fantôme d'agar. Les résultats de cette expérience sont illustrés sur la figure 9 qui représente un diamètre de tache à 50 % d'intensité lumineuse en fonction de l'épaisseur du fantôme d'agar pour des diodes électroluminescentes (LED) de différentes longueurs d'onde.

Comme le lecteur appréciera sur la figure 9, le diamètre de la tache augmente avec l'épaisseur du fantôme, suggérant une diminution de la précision de la position de la source lumineuse de type LED.

L'association d'une diode électroluminescente (LED) émettant un rayonnement lumineux à une longueur d'onde de 1050nm et d'un fantôme d'épaisseur 16.16mm donne une tache de diamètre suffisamment petit (19.40mm) pour distinguer trois taches dans le cas d'un dispositif médical implantable 1 comportant trois diodes électroluminescentes réparties à égale distance de la borne de connexion 113 et séparées entre elles d'une distance de 25 millimètres).

A titre indicatif, des essais ont été réalisés avec un dispositif médical implantable 1 incluant un repère de positionnement comportant trois diodes électroluminescentes (LED) :
- émettant un rayonnement lumineux à la longueur d'onde de 1050 nm (infrarouge),
- séparées les unes des autres d'une distance de 25 millimètres,
- réparties à égale distance de la borne de connexion.

L'unité de localisation a permis de localiser la position de la borne de connexion 113 :
- avec une précision de 2,4 mm dans 87 % des cas, et
- avec une précision de 3,2 mm dans 100 % des cas,
pour 30 essais réalisés avec deux fantômes plans d'épaisseurs 9 mm et 14 mm et un fantôme non plan ayant une épaisseur variable comprise entre 6 mm et 13 mm.

Bien entendu, le lecteur appréciera que des sources lumineuses émettant des rayonnements lumineux à des longueurs d'onde plus importantes peuvent être utilisés pour minimiser le phénomène de diffusion à travers le cuir chevelu du patient.

En outre, le lecteur appréciera que des sources lumineuses émettant des rayonnements lumineux à des longueurs d'onde différentes les unes des autres peuvent être utilisés dans le repère de positionnement. Ceci permet de détecter le centre de chaque tache (et donc la position de chaque source lumineuse), même en cas de superposition des taches produites par des sources lumineuse différentes.

### 2.5.2. Solution à résonance électromagnétique

Pour détecter la position de la borne de connexion 113, la solution électromagnétique utilise une variation d'impédance lorsque deux circuits résonants interfèrent.

Pour la mise en œuvre de la solution électromagnétique, plusieurs circuits électriques résonnants sont disposés dans l'unité de localisation et dans le dispositif médical 1 implantable.

Plus précisément :
- le repère de positionnement du dispositif médical implantable comprend :
   ∘ un circuit électrique passif résonnant incluant une bobine entourant la borne de connexion 113, ou
   ∘ plusieurs circuits électriques passifs résonnants incluant chacun une (ou plusieurs) bobine(s) respective(s), lesdites bobines étant réparties autour de la borne de connexion 113 à égale distance de celle-ci,
- l'unité de localisation comprend un (ou plusieurs) circuit(s) électrique(s) actifs résonnant(s) incluant chacun une (ou plusieurs) bobine(s) disposée (réparties) sur une surface destinée à venir en contact avec le cuir chevelu du patient.

Le fait que le repère de positionnement comprenne un circuit électrique résonnant incluant une bobine - plutôt qu'un aimant permanent - permet de rendre l'appareil de traitement selon l'invention compatible avec les techniques d'imagerie par résonance magnétique (IRM).

Si la solution optique permet de détecter la position de la borne de connexion avec une grande précision (±1.6mm), la solution électromagnétique permet quant à elle de disposer d'une information concernant l'orientation de la borne de connexion, ce qui facilite encore le geste de l'opérateur pour insérer l'extrémité de l'aiguille transdermique 32 dans la borne de connexion113.

### 2.5.2.1. Principe

La solution électromagnétique utilise la variation d'impédance lorsque deux circuits résonants sont suffisamment proches pour interférer, ou lorsque l'impédance d'un circuit comprenant une bobine est modifiée par la présence d'un métal ou d'un aimant.

Le principe de la solution électromagnétique est d'utiliser la variation causée par l'induction électromagnétique lors du déplacement de l'unité de localisation au-dessus du dispositif médical implantable pour localiser la position de la borne de connexion 113.

Plus précisément, une bobine primaire (contenue dans l'unité de localisation) induit un champ électromagnétique dirigé vers une bobine secondaire (contenue dans le dispositif médical implantable). Lorsque les bobines primaire et secondaire sont rapprochées, la transmission de puissance à la bobine secondaire provoque une perte dans le circuit électrique résonant incluant la bobine primaire. En détectant la variation correspondante dans le circuit primaire, l'emplacement de la borne de connexion peut être déduit.

Le principe de la solution électromagnétique est bien connu et a été décrit dans de nombreux documents, tels que US 5 697 377.

### 2.5.2.2. Expérimentation et résultats

Une expérience a été réalisée pour étudier la précision dans la détection de la borne de connexion à l'aide de la solution électromagnétique.

Le dispositif médical implantable comprenait un circuit électrique résonnant comportant :
- une bobine de 5 tours ayant un diamètre de 2.5 cm intégrée à la collerette 132 de la pièce de fixation 13,
- une capacité montée en parallèle dont la valeur était choisie pour obtenir un circuit résonant ayant une fréquence de résonnance de 16.5 MHz.

Ce dispositif médical implantable a été inséré dans un fantôme d'agar imitant le comportement du cuir chevelu. Ce fantôme d'agar était composé :
- de 80 cl d'eau,
- de 20g de glycine,
- de 0,6 g de chlorure de sodium (NaCl), et de
- de 1.6 g de poudre de gélose.

Deux types d'unités de localisation ont été testés :
- la première unité de localisation comprenait une bobine identique à celle contenue dans le circuit résonant du dispositif médical implantable et un condensateur monté en parallèle et dont la valeur était choisie pour obtenir un circuit résonant ayant une fréquence de résonnance de 16.5 MHz (fréquence de résonance identique à la fréquence de résonance du circuit résonant contenu dans le dispositif médical implantable),
- la deuxième unité de localisation comprenait une bobine en spirale (forme intéressante pour la transmission d'énergie) de 18 tours et de diamètre extérieur environ égale à 3 cm, et une capacité ajustée pour avoir une fréquence de résonnance égale à 16.5 MHz (fréquence de résonance du circuit résonant contenu dans le dispositif médical implantable).

Le fait que les bobines (contenues dans le dispositif médical implantable et dans l'unité de localisation) soient respectivement associées à des capacités a permis d'obtenir des circuits résonants à la même fréquence dans le dispositif médical d'une part et dans l'unité de localisation d'autre part.

Les inventeurs ont en effet découvert que l'utilisation de circuits résonnants permettait :
- d'améliorer l'efficacité du couplage entre le dispositif médical et l'unité de localisation, et
- d'améliorer la capacité de détection de ce couplage, par rapport au solutions basés sur l'utilisation d'une bobine et d'un aimant par exemple.

La précision dans la détection de la position de la borne de connexion était inférieure à celle obtenue avec la solution optique.

Toutefois, les inventeurs ont découvert que la combinaison des deux solutions permettait de faciliter la connexion de l'aiguille transdermique à la borne de connexion 113, notamment par la fourniture d'une information précise sur la position de la borne de connexion (solution optique) et sur son orientation (solution électromagnétique).

En outre, l'utilisation de circuits résonants telle que proposée avec la solution électromagnétique permet d'alimenter électriquement le dispositif médical implantable par induction. Il n'est donc plus nécessaire que des batteries soient intégrées à celui-ci pour la mise en œuvre de la solution optique.

### 3. Unité de localisation

Comme indiqué précédemment, l'unité de localisation peut être intégrée aux moyens de connexion ou être séparée de ceux-ci. En particulier, l'unité de localisation peut être intégrée :
- aux moyens de connexion, ou
- à un outil destiné à être fixé sur les moyens de connexion, ou
- à un outil intermédiaire totalement indépendant des moyens de connexion et permettant à l'opérateur de localiser l'implant.

Dans une variante de réalisation, l'unité de localisation est intégrée à un outil incluant une poignée de préemption et l'unité de localisation comportant des capteurs de proximité aptes à interférer avec le repère de positionnement contenu dans le dispositif médical implantable.

Les capteurs de proximité évaluent l'alignement de la poignée avec le dispositif médical, afin de détecter la position de la borne de connexion et ainsi faciliter l'insertion de l'aiguille transdermique dans celle-ci.

En fonctionnement, l'opérateur peut marquer l'emplacement d'insertion de l'aiguille sur la peau avec un stylo-feutre, puis retirer l'outil intermédiaire et perforer la peau sur la marque.

### 4. Exemple de mise en œuvre

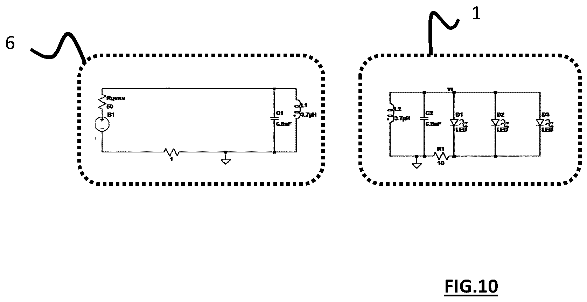

En référence à la figure 10 on a illustré un exemple de mise en œuvre de l'appareil de traitement. Plus précisément, on a illustré des exemples de circuits électroniques intégrés dans le dispositif médical 1 d'une part, et dans l'unité de localisation 6 d'autre part.

Le dispositif médical comprend un circuit résonant LC (incluant une bobine L2 de 3.7µH montée en parallèle d'une capacité C2 de 6.8nF), et trois sources lumineuses D1, D2, D3 de type LED montées chacune en parallèle du circuit résonant LC. Ce circuit électronique est intégré dans la pièce de fixation, en particulier dans la collerette.

L'unité de localisation 6 comprend une caméra (non représentée), un générateur B, une résistance R_{gene} montée en série avec le générateur B, et un circuit résonant LC (incluant une bobine L1 de 3.7µH montée en parallèle d'une capacité C1 de 6.8nF) connecté au générateur B.

### 5. Principe de fonctionnement

Le principe de fonctionnement de l'appareil de traitement décrit précédemment est le suivant.

A chaque nouvelle séance de traitement, un opérateur déplace l'unité de localisation à proximité de la tête du patient.

Pour une meilleure détection ou pour limiter l'énergie à fournir au circuit résonnant, il est possible de faire glisser l'unité de localisation sur le cuir chevelu du patient. En effet pour alimenter en énergie électrique les sources lumineuses par induction, il est préférable de limiter la distance entre le circuit résonant de l'unité de localisation et le circuit résonnant contenu dans le dispositif médical.

Pour éviter la contamination, l'unité de localisation peut être recouverte d'une enveloppe stérile, telle qu'une enveloppe du type « *Ultrasound Probe Cover* » développée par la société CIVCO.

L'unité de localisation est déplacée sur la tête du patient. Lorsqu'une perturbation d'impédance maximum est détectée au niveau de l'unité de localisation, celle-ci émet un signal (visuel ou sonore, etc.) pour avertir l'opérateur de la position de la borne de connexion. L'énergie électromagnétique transmise par le circuit résonant de l'unité de localisation permet d'alimenter par induction les sources lumineuses du repère de positionnement.

Le détecteur de l'unité de localisation acquiert une image des taches lumineuses produites par les sources lumineuses contenues dans le dispositif médical. Le calculateur de l'unité de localisation calcule les centres des taches (représentatifs des positions des sources lumineuses), et estime la position du barycentre des centres des taches (représentatif de la position de la borne de connexion). Ces différentes informations peuvent être affichées sur les moyens d'affichage.

L'opérateur peut alors prendre un marqueur (tel qu'un feutre) et marquer la position du barycentre qui correspond à la position du point à piquer avec l'aiguille transdermique.

Le lecteur aura compris que de nombreuses modifications peuvent être apportées à l'appareil de traitement décrit précédemment sans sortir des enseignements présentés ici.

Par exemple, même si l'appareil était présenté en référence à un dispositif médical implantable permettant le traitement et/ou l'imagerie d'une zone cérébrale d'intérêt par ultrasons, il est bien évident que :
- Le dispositif médical implantable peut être configuré pour le traitement et/ou l'imagerie d'un autre tissu d'intérêt du corps humain ou animal,
- Le dispositif médical implantable peut comprendre une unité de traitement basé sur une technologie autre que les ultrasons.

Par ailleurs, les différents composants permettant la mise en œuvre des solutions optique et électromagnétique présentées ci-dessus peuvent être inversés dans le dispositif médical implantable et dans l'unité de localisation. Notamment, le dispositif médical implantable peut intégrer une caméra et un circuit électrique résonant actif, tandis que l'unité de localisation intègre des sources lumineuses et un circuit résonant passif.

## Revendications

1. Appareil de traitement d'une pathologie comprenant :
- un dispositif implantable (1) au niveau d'une ouverture ménagée dans la boîte crânienne d'un patient, le dispositif implantable (1) comportant une unité de traitement (11) ayant des faces supérieure et inférieure et incluant une borne de connexion électrique (114) s'étendant sur la face supérieure,
- une unité de commande (2) distante pour déterminer et contrôler des paramètres de fonctionnement du dispositif implantable (1), et lui délivrer de l'électricité,
- des moyens de connexion électrique (3) pour le raccordement électrique de l'unité de traitement (11) à l'unité de commande distante par l'intermédiaire de la borne de connexion électrique (114),
***caractérisé en ce que*** le dispositif implantable comprend un repère de positionnement pour faciliter la détection de la position de la borne de connexion électrique (114), ledit repère de positionnement comprenant au moins deux sources lumineuses, telles que des diodes électroluminescentes, aptes à émettre chacune un rayonnement lumineux vers l'extérieur de la face supérieure, la borne de connexion électrique (114) s'étendant entre lesdites diodes électroluminescentes.

2. Appareil selon la revendication 1, ***dans lequel*** la longueur d'onde du rayonnement lumineux émis par chaque diode électroluminescente est compris entre 600 et 1600 nanomètres, préférentiellement comprise entre 850 et 1250 nanomètres, et encore plus préférentiellement compris entre 950 et 1100 nanomètres, notamment de l'ordre de 1050 nanomètres.

3. Appareil selon l'une quelconque des revendications 1 ou 2, ***dans lequel*** la distance entre les diodes électroluminescentes est comprise entre 15 et 60 millimètres, de préférence comprise entre 20 et 40 millimètres, notamment de l'ordre de 25 millimètres.

4. Appareil selon l'une quelconque des revendications 1 à 3, dans lequel la distance entre chaque diode électroluminescente et la borne de connexion est comprise entre 9 et 35 millimètres, de préférence comprise entre 12 et 25 millimètres, notamment de l'ordre de 15 millimètres.

5. Appareil selon l'une quelconque des revendications 1 à 4, ***dans lequel*** chaque diode électroluminescente est configurée pour émettre un rayonnement lumineux ayant une longueur d'onde respective différente des longueurs d'onde des autres diodes électroluminescentes.

6. Appareil selon l'une quelconque des revendications 1 à 5, ***dans lequel*** chaque diode électroluminescente est configurée pour émettre un rayonnement lumineux ayant une intensité respective différente des intensités des autres diodes électroluminescentes.

7. Appareil selon l'une quelconque des revendications 1 à 6, ***dans lequel*** le repère de positionnement comprend en outre au moins un circuit électrique passif résonnant.

8. Appareil selon la revendication 7, ***dans lequel*** ledit et au moins un circuit électrique passif résonnant est configuré pour interagir avec un circuit électrique résonant actif intégré à une unité de localisation, ladite interaction permettant d'alimenter le dispositif médical implantable en énergie électrique par induction.

9. Appareil selon la revendication 8, ***dans lequel*** ledit et au moins un circuit électrique passif résonnant comprend une bobine fonctionnellement couplée à un condensateur, ladite bobine s'étendant autour de la borne de connexion électrique (114).

10. Appareil selon l'une quelconque des revendications 7 à 9, ***dans lequel*** ledit et au moins un circuit électrique passif résonnant est configuré de sorte que la fréquence de résonnance dudit et au moins un circuit électrique passif résonnant est comprise entre 10MHz et 50 MHz.

11. Appareil selon l'une quelconque des revendications 1 à 10, ***dans lequel*** le dispositif implantable (1) comprend :
- une plaque support incluant des première et deuxième faces opposées, l'unité de traitement (11) étant destinée à être montée sur la première face de la plaque support (12),
- une pièce de fixation (13) destinée à être positionnée sur la deuxième face de la plaque support (12) et étant configurée pour plaquer l'unité de traitement (11) contre la première face de la plaque support (12) lorsque l'unité de traitement (11), la plaque support (12) et la pièce de fixation (13) sont assemblées,
***et dans lequel*** ladite pièce de fixation inclut le repère de positionnement.

12. Appareil selon la revendication 11, ***dans lequel* :**
- la plaque support (12) comprend un orifice traversant (122),
- la borne de connexion électrique (114) comprend un pion (1141) s'étendant en saillie vers l'extérieur de l'unité de traitement, le pion étant destiné à être positionné dans l'orifice traversant de la plaque support,
- la pièce de fixation (13) comprend :
∘ un conduit (131) adapté pour recevoir au moins une portion du pion (1141), et
∘ une collerette périphérique (132) s'étendant perpendiculairement à un axe longitudinal du conduit (131), la collerette (132) étant destinée à être positionnée sur la deuxième face de la plaque support (12),
***et dans lequel*** ladite collerette périphérique inclut le repère de positionnement.

## Patentansprüche

1. Apparat zur Behandlung einer Erkrankung, umfassend:
- eine implantierbare Vorrichtung (1) an einer Öffnung in der Schädelhöhle eines Patienten, wobei die implantierbare Vorrichtung (1) eine Behandlungseinheit (11) mit einer Oberseite und einer Unterseite und eine elektrische Anschlussklemme (114) umfasst, die sich auf der Oberseite erstreckt,
- eine entfernte Steuereinheit (2) zum Bestimmen und Steuern der Betriebsparameter der implantierbaren Vorrichtung (1) und zum Versorgen dieser mit Strom,
- elektrische Anschlussmittel (3) zum elektrischen Verbinden der Behandlungseinheit (11) mit der entfernten Steuereinheit über die elektrische Anschlussklemme (114),
**dadurch gekennzeichnet, dass** die implantierbare Vorrichtung eine Positionierungsmarkierung umfasst, um das Erkennen der Position der elektrischen Anschlussklemme (114) zu erleichtern, wobei die Positionierungsmarkierung mindestens zwei Lichtquellen wie beispielsweise Leuchtdioden umfasst, die imstande sind, jeweils eine Lichtstrahlung nach außen von der Oberseite senden zu können, wobei sich die elektrische Anschlussklemme (114) zwischen den Leuchtdioden erstreckt.

2. Apparat nach Anspruch 1, wobei die Wellenlänge der von jeder Leuchtdiode gesendeten Lichtstrahlung zwischen 600 und 1600 Nanometern, vorzugsweise zwischen 850 und 1250 Nanometern und noch bevorzugter zwischen 950 und 1100 Nanometern, insbesondere in der Größenordnung von 1050 Nanometern, liegt.

3. Apparat nach einem der Ansprüche 1 oder 2, wobei der Abstand zwischen den Leuchtdioden zwischen 15 und 60 Millimetern, vorzugsweise zwischen 20 und 40 Millimetern, insbesondere in der Größenordnung von 25 Millimetern, liegt.

4. Apparat nach einem der Ansprüche 1 bis 3, wobei der Abstand zwischen jeder Leuchtdiode und der Anschlussklemme zwischen 9 und 35 Millimetern, vorzugsweise zwischen 12 und 25 Millimetern, insbesondere in der Größenordnung von 15 Millimetern, liegt.

5. Apparat nach einem der Ansprüche 1 bis 4, wobei jede Leuchtdiode ausgelegt ist, um eine Lichtstrahlung mit einer jeweiligen Wellenlänge zu senden, die sich von den Wellenlängen der anderen Leuchtdioden unterscheidet.

6. Apparat nach einem der Ansprüche 1 bis 5, wobei jede Leuchtdiode ausgelegt ist, um eine Lichtstrahlung mit einer Stärke zu senden, die sich von den Stärken der anderen Leuchtdioden unterscheidet.

7. Apparat nach einem der Ansprüche 1 bis 6, wobei die Positionsmarkierung ferner mindestens eine passive elektrische Resonanzschaltung umfasst.

8. Apparat nach Anspruch 7, wobei die und mindestens eine passive elektrische Resonanzschaltung ausgelegt ist, um mit einer aktiven elektrischen Resonanzschaltung, die in eine Ortungseinheit integriert ist, zu interagieren, wobei die Interaktion es ermöglicht, die implantierbare medizinische Vorrichtung durch Induktion mit elektrischer Energie zu versorgen.

9. Apparat nach Anspruch 8, wobei die und mindestens eine passive elektrische Resonanzschaltung eine Spule umfasst, die funktionell mit einem Kondensator gekoppelt ist, wobei sich die Spule um die elektrische Anschlussklemme (114) erstreckt.

10. Apparat nach einem der Ansprüche 7 bis 9, wobei die und mindestens eine passive elektrische Resonanzschaltung derart ausgelegt ist, dass die Resonanzfrequenz der und mindestens einen passiven Resonanzschaltung zwischen 10 MHz und 50 MHz liegt.

11. Apparat nach einem der Ansprüche 1 bis 10, wobei die implantierbare Vorrichtung (1) umfasst:
- eine Trägerplatte mit einer ersten und einer zweiten gegenüberliegenden Seite, wobei die Behandlungseinheit (11) dazu bestimmt ist, auf der ersten Seite der Trägerplatte (12) angebracht zu sein,
- ein Befestigungsteil (13), das dazu bestimmt ist, auf der zweiten Seite der Trägerplatte (12) positioniert zu sein und ausgelegt ist, um die Behandlungseinheit (11) gegen die erste Seite der Trägerplatte (12) zu drücken, wenn die Behandlungseinheit (11) die Trägerplatte (12) und das Befestigungsteil (13) miteinander verbunden sind,
und wobei das Befestigungsteil die Positionierungsmarkierung umfasst.

12. Apparat nach Anspruch 11, wobei
- die Trägerplatte (12) eine Durchgangsöffnung (122) umfasst,
- die elektrische Anschlussklemme (114) einen Zapfen (1141) umfasst, der sich nach außen aus der Behandlungseinheit heraus hervorstehend erstreckt, wobei der Zapfen dazu bestimmt ist, in der Durchgangsöffnung der Trägerplatte positioniert zu werden,
- das Befestigungsteil (13) umfasst:
o einen Kanal (131), der zur Aufnahme mindestens eines Abschnitts des Zapfens (1141) ausgelegt ist, und
o einen umlaufenden Kragen (132), der sich senkrecht zu einer Längsachse des Kanals (131) erstreckt, wobei der Kragen (132) dazu bestimmt ist, auf der zweiten Seite der Trägerplatte (12) positioniert zu werden,
und wobei der umlaufende Kragen die Positionierungsmarkierung umfasst.

## Claims

1. An apparatus for treating a pathology comprising:
- a device (1) implantable at an opening made in the cranium of a patient, the implantable device (1) including a treatment unit (11) having upper and lower faces and including an electrical connection terminal (114) extending on the upper face,
- a remote-control unit (2) for determining and controlling operating parameters of the implantable device (1), and delivering electricity thereto,
- electrical connection means (3) for electrically connecting the treatment unit (11) to the remote-control unit via the electrical connection terminal (114),
***characterised in that*** the implantable device comprises a positioning indicator in order to facilitate detection of the position of the electrical connection terminal (114), said positioning indicator comprising at least two light sources, such as light-emitting diodes, each capable of emitting light radiation towards the outside of the upper face, the electrical connection terminal (114) extending between said light-emitting diodes.

2. The apparatus according to claim 1, ***wherein*** the wavelength of the light radiation emitted by each light-emitting diode is comprised between 600 and 1600 nanometres, preferably comprised between 850 and 1250 nanometres, and even more preferably comprised between 950 and 1100 nanometres, in particular of the order of 1050 nanometres.

3. The apparatus according to any one of claims 1 or 2, ***wherein*** the distance between the light-emitting diodes is comprised between 15 and 60 millimetres, preferably comprised between 20 and 40 millimetres, in particular of the order of 25 millimetres.

4. The apparatus according to any one of claims 1 to 3, ***wherein*** the distance between each light-emitting diode and the connection terminal is comprised between 9 and 35 millimetres, preferably comprised between 12 and 25 millimetres, in particular of the order of 15 millimetres.

5. The apparatus according to any one of claims 1 to 4, ***wherein*** each light-emitting diode is configured to emit light radiation having a respective wavelength different from the wavelengths of the other light-emitting diodes.

6. The apparatus according to any one of claims 1 to 5, ***wherein*** each light-emitting diode is configured to emit light radiation having a respective intensity different from the intensities of the other light-emitting diodes.

7. The apparatus according to any one of claims 1 to 6, ***wherein*** the positioning indicator further comprises at least one resonant passive electrical circuit.

8. The apparatus according to claim 7, ***wherein*** said and at least one resonant passive electrical circuit is configured to interact with a resonant active electrical circuit integrated into a location unit, said interaction allowing to supply the implantable medical device with electrical energy by induction.

9. The apparatus according to claim 8, ***wherein*** said and at least one resonant passive electrical circuit comprises a coil functionally coupled to a capacitor, said coil extending around the electrical connection terminal (114).

10. The apparatus according to any one of claims 7 to 9, ***wherein*** said and at least one resonant passive electric circuit is configured so that the resonance frequency of said and at least one resonant passive electric circuit is comprised between 10 MHz and 50 MHz.

11. The apparatus according to any one of claims 1 to 10, ***wherein*** the implantable device (1) comprises:
- a support plate including first and second opposite faces, the treatment unit (11) being intended to be mounted on the first face of the support plate (12),
- a fixing part (13) intended to be positioned on the second face of the support plate (12) and being configured to press the treatment unit (11) against the first face of the support plate (12) when the treatment unit (11), the support plate (12) and the fixing part (13) are assembled,
***and wherein*** said fixing part includes the positioning indicator.

12. The apparatus according to claim 11, ***wherein*:**
- the support plate (12) comprises a through orifice (122),
- the electrical connection terminal (114) comprises a pin (1141) projecting outwards from the treatment unit, the pin being intended to be positioned in the through orifice of the support plate,
- the fixing part (13) comprises:
∘ a conduit (131) adapted to receive at least a portion of the pin (1141), and
∘ a peripheral flange (132) extending perpendicular to a longitudinal axis of the conduit (131), the flange (132) being intended to be positioned on the second face of the support plate (12),
***and wherein*** said peripheral flange includes the positioning indicator.
